# EUROPÄISCHE PATENTSCHRIFT

(11) **EP 0 703 919 B1**
(45) Veröffentlichungstag und Bekanntmachung des Hinweises auf die Patenterteilung: **02.09.1998**
(21) Anmeldenummer: 94918879.1
(22) Anmeldetag: 17.06.1994
(51) Int. Cl.: C07F 9/38, A61K 31/66, C07F 9/572, C07F 9/59, C07F 9/655, C07F 9/6553, C07F 9/6533

(54) **PHOSPHONOBERNSTEINSÄUREDERIVATE UND IHRE VERWENDUNG ALS ARZNEIMITTEL**
PHOSPHOSUCCINIC ACID DERIVATIVES AND THEIR USE AS MEDICAMENTS
DERIVES D'ACIDE PHOSPHOSUCCINIQUE ET LEUR UTILISATION COMME MEDICAMENTS

(30) Priorität: 18.06.1993 DE 4320223
(43) Veröffentlichungstag der Anmeldung: 03.04.1996
(73) Patentinhaber: BOEHRINGER MANNHEIM GMBH, 68298 Mannheim (DE)
(72) Erfinder: TSAKLAKIDIS, Christos, D-69469 Weinheim (DE); ESSWEIN, Angelika, D-78224 Singen (DE); ZIMMERMANN, Gerd, D-68309 Mannheim (DE); BAUSS, Frieder, D-67245 Lambsheim (DE)
(86) Internationale Anmeldenummer: EP9401980
(87) Internationale Veröffentlichungsnummer: WO9500522

(56) Entgegenhaltungen:
- WO-A-93/12122
- WO-A-93/24131
- DE-A- 2 360 797

## Beschreibung

Die vorliegende Erfindung betrifft neue Phosphonobernsteinsäurederivate, Verfahren zu deren Herstellung sowie Arzneimittel, die diese Substanzen enthalten.

Es wurde nun gefunden, daß Phosphonobernsteinsäurederivate der vorliegenden Erfindung eine ausgezeichnete Wirkung auf den Calciumstoffwechsel zeigen und damit zur breiten Behandlung von Calciumstoffwechselstörungen geeignet sind. Sie lassen sich vor allem sehr gut dort einsetzen, wo der Knochenauf- und abbau gestört ist, d.h. sie sind geeignet zur Behandlung von Erkrankungen des Skelettsystems wie z.B. Osteoporose, Morbus Paget, Morbus Bechterew u.a..

Aufgrund dieser Eigenschaften finden sie aber auch Verwendung in der Therapie der Urolithiasis und zur Verhinderung heterotoper Ossifikationen. Durch ihre Beeinflussung des Calciumstoffwechsels bilden sie weiterhin eine Grundlage für die Behandlung der rheumatoiden Arthritis, der Osteoarthritis und der degenerativen Arthrose.

Gegenstand der vorliegenden Erfindung sind Verbindungen der allgemeinen Formel I, 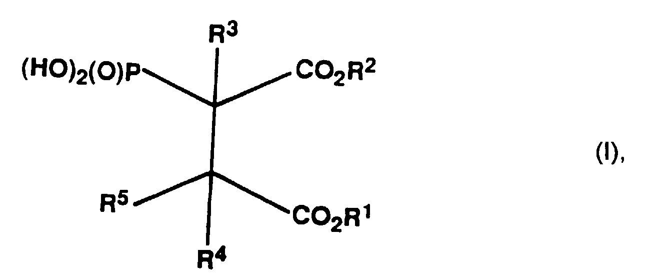 in der
- R¹, R²: unabhängig voneinander jeweils Wasserstoff, niederes Alkyl, Cycloakyl oder Arylmethyl sein können,
- R³, R⁴: unabhängig voneinander jeweils Wasserstoff, niederes Alkyl, oder eine der Gruppen -OR⁶ oder -NR⁷R⁸ sein können, oder zusammen mit den Atomen, an die sie gebunden sind, einen fünf- bis siebengliedrigen carbocyclischen oder einen eins bis zwei Heteroatome enthaltenden heterocyclischen Ring bilden können,
- R⁵: niederes Alkenyl, Cycloalkyl, Cycloalkenyl, monocyclisches Arylalkyl, bicyclisches Aryl, Biaryl, oder eine Gruppe der Formeln a) oder b) bedeutet, oder zusammen mit R⁴ und dem Kohlenstoffatom an dem es gebunden ist, einen gegebenenfalls substituierten fünf- bis siebengliedrigen carbocvclischen oder heterocyclischen Ring bildet,

a) R⁹-X-alk- 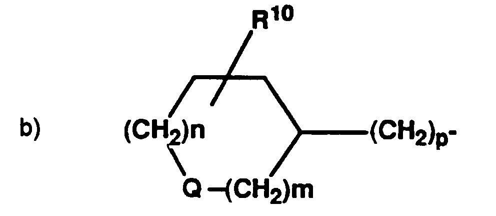
- R⁶: Wasserstoff, niederes Alkyl oder Arylmethyl bedeutet,
- R⁷, R⁸: unabhängig voneinander jeweils Wasserstoff oder niederes Alkyl bedeuten, oder zusammen mit dem Stickstoffatom, an das sie gebunden sind, einen fünf- bis sechsgliedrigen heterocyclischen Ring bilden,
- R⁹: Wasserstoff, niederes Alkyl, niederes Alkenyl, Cycloalkyl, Cycloalkenyl, mono- oder bicyclisches Aryl, Biaryl bedeutet,
- R¹⁰: eine Gruppe der Formel a) bedeutet,
- X: Valenzstrich, Sauerstoff oder Schwefel bedeutet,
- Q: Sauerstoff, Schwefel oder Stickstoff bedeutet,
- alk: einen Valenzstrich, eine Methylen-, eine gesättigte oder ungesättigte Alkylenkette mit 2 - 6 Kohlenstoffatomen bedeutet,
- n =: 0 - 3,
- m =: 0 - 2 und
- p =: 0 - 5 bedeutet,
sowie deren pharmakologisch unbedenklichen Salze und optische Isomeren, wobei für den Fall, daß
- R³: Wasserstoff oder C₁-C₃-Alkyl und R⁴ Wasserstoff bedeutet, R⁵ nicht Wasserstoff, Hydroxy, Methoxy, C₁-C₃-Alkyl oder Aryl sein darf,
und für den Fall, daß
- X: Sauerstoff oder Schwefel und R⁴ eine der Gruppen OR⁶ oder NR⁷R⁸ bedeuten, alk kein Valenzstrich sein darf, und mit Ausnahme der Verbindungen
2-Phosphono-3-(pyrrolidin-2-yl )-bernsteinsäure,
2-Phosphono-3-(pyrrolidin-3-yl)-bernsteinsäure,
2-Phosphono-3-(pyrrolidin-2-yl-methyl)-bernsteinsäure,
2-Phosphono-3-(pyrrolidin-3-yl-methyl)-bernsteinsäure,
2-Phosphono-3-[2-(pyrrolidin-2-yl)ethyl]-bernsteinsäure,
2-Phosphono-3-[2-(pyrrolidin-3-yl)ethyl]-bernsteinsäure,
2-Phosphono-3-[4-(pyrrolidin-2-yl)butyl]-bernsteinsäure.

In DE-A-23 60 797 sind bereits Phosphonobernsteinsäure-Derivate der Formel I, in der R³ Wasserstoff oder C₁-C₃-Alkyl, R⁴ Wasserstoff und R⁵ Wasserstoff oder C₁-C₃-Alkyl bedeuten, zur Beeinflussung der Ablagerung und Auflösung von schwerlöslichen Calciumsalzen beschrieben. In der nicht vorveröffentlichten, aber prioritätsälteren Anmeldung WO-A-93 12122 sind die im Disclaimer aufgeführten Verbindungen namentlich angeführt.

Weitere Verbindungen der Formel I ohne Angabe einer möglichen Verwendung als Arzneimittel sind in den folgenden Chemical Abstract-Auszügen beschrieben:
Verbindungen mit R₃ = H oder CH₃; R⁴ = H und R⁵ = OCH₃ in CA, 105(5):42932 x und CA, 104(1):5939.
Verbindungen mit R³ = H; R⁴ = H und R⁵ = Phenyl, gegebenenfalls substituiert in CA 115(23):255757 n und CA106(21):176504 p.

Niederes Alkyl soll in allen Fällen eine geradkettige oder verzweigte C₁-C₆-Alkylgruppe wie z.B. Methyl, Ethyl, Propyl, Isopropyl, Butyl, Isobutyl, Pentyl oder Hexyl, insbesondere Methyl, Ethyl, Propyl, Isobutyl und Pentyl darstellen.

Niederes Alkenyl bedeutet ungesättigte Reste mit 3 - 6 Kohlenstoffatomen wie z.B. Allyl, But-2-enyl, Hexa-2,4-dienyl, vor allem Allyl.

Cycloalkyl bedeutet einen gegebenenfalls substituierten 3 - 7-gliedrigen Ring, wie den Cyclopropyl-, Cyclobutyl-, Cyclopentyl-, Cyclohexyl- oder den Cycloheptylring, insbesondere den Cyclopropyl, Cyclopentyl- und Cyclohexylring. Diese Cycloalkylreste können ein- oder zweifach durch C₁-C₆-Alkylgruppen, vorzugsweise die Methyl-, Ethyl- oder Isopropylgruppe, sowie durch Hydroxy-, Methoxy-, Benzyloxy-, Amino-, Methylamino-, Dimethylamino-, Benzylaminogruppen oder durch Chlor oder Brom substituiert sein.

Cycloalkenyl bedeutet einen gegebenenfalls substituierten Cyclopentenyl-, Cyclohexenyl- oder Cycloheptenylring. Diese Ringe können ein- oder zweifach durch C₁ -C₆-Alkylgruppne, vorzugsweise die Methyl, Ethyl oder Isopropylgruppe, sowie Chlor, Brom oder Hydroxy-, Methoxy-, Benzyloxy-, Amino-, Methylamino-, Dimethylamino- oder Benzylaminogruppen substituiert sein.

Falls die Reste R³ und R⁴ zusammen mit den Kohlenstoffatomen an die sie gebunden sind einen carbocyclischen oder heterocyclischen Ring bilden, handelt es sich um einen gesättigten oder ungesättigten 5-7-gliedrigen Ring, wie den Cyclopentyl-, Cyclohexyl-, Cycloheptyl-, Pyrrolidin-, Piperidin-, Azepin-, Tetrahydrofuran-, Tetrahydropyran-, Morpholin-, Dioxan-, Cyclopentenyl-, Cyclohexenyl-, Pyrrolin-, Dihydrofuran- oder den Dihydropyranring, insbesondere den Cyclopentyl-, Cyclohexyl-, Tetrahydrofuran-, Morpholin-, Cyclohexenyl-, oder den Dihydropyranring.

Falls R4 und R5 zusammen mit dem Kohlenstoffatom an dem sie gebunden sind, einen carbocyclischen oder heterocyclischen Ring bilden, handelt es sich um einen gesättigten oder ungesättigten 5-7-gliedrigen Ring, wie den Cyclopentyl-, Cyclohexyl-, Cycloheptyl, Pyrrolidin-, Piperidin-, Azepin-,Tetrahydrofuran-, Tetrahydropyran-, Morpholin-, Dioxan-, Cyclopentenyl-, Cyclohexenyl-, Pyrrolin-, Dihydrofuran-oder den Dihydropyranring, insbesondere den Cyclopentyl-, Cyclohexyl-, Cyclohexenyl-, Pyrrolidin-, Piperidin-, Tetrahydrofuran-, Tetrahydropyran- oder den Morpholinring.

Die carbocyclischen und heterocyclischen Ringe können gegebenenfalls ein oder zweifach durch C₁ -C₆-Alkylgruppen, vorzugsweise die Methyl-, Ethyl- oder Isopropylgruppe, sowie durch Chlor, Brom oder Hydroxy-, Methoxy-, Benzyloxy-, Amino-, Methylamino-, Dimethylamino- oder Benzylaminogruppen substituiert sein.

Aryl bedeutet in der Regel den gegebenenfalls ein- oder mehrfach substituierten Phenylrest.

Bicyclisches Aryl bedeutet in der Regel einen gegebenenfalls ein- oder mehrfach substituierten Indan-, Naphthalin- oder Anthracenrest, vorzugsweise den Naphthalinrest.

Biaryl bedeutet in der Regel einen gegebenenfalls ein- oder mehrfach substituierten Biphenylrest.

Aryl-, bicyclische Aryl- und Biarylreste können gegebenenfalls ein- oder mehrfach durch C₁-C₆-Alkylgruppen, vorzugsweise die Methyl-, Ethyl- oder Isopropylgruppe, sowie durch Chlor, Brom, Fluor, oder Hydroxy-, Alkoxy wie z. B. Methoxy-, Benzyloxy-, Acetyloxy-, Carboxy-, Ethoxycarbonyl-, Aminocarbonyl-, Methylaminocarbonyl-, Dimethylaminocarbonyl-, Cyano-, Amino-, Methylamino-, Dimethylamino-, Benzyl-amino-, Acetylamino-, Benzoyl- amino- und Amidinogruppen substituiert sein.

Arylalkyl bedeutet in der Regel einen unsubstituierten oder ein- oder mehrfach substituierten Benzyl-, Phenethyl-, Phenylpropyl-, Phenylbutyl- oder Phenylpentylrest, vorzugsweise einen Benzyl-, Phenethyl- oder Phenylpentylrest. Als Substituenten kommen C₁ -C₆-Alkylreste, vorzugsweise Methyl-, Ethyl- oder Isopropyl, sowie Chlor, Brom, Fluor, oder Hydroxy-, Methoxy-, Benzyloxy-, Acetyloxy-, Carboxy-, Ethoxy-carbonyl-, Aminocarbonyl-, Methylaminocarbonyl-, Dimethylaminocarbonyl-, Cyano-, Amino-, Methylamino-, Dimethylamino-, Benzylamino-, Acetylamino-, Benzoylamino- und Amidinogruppen infrage.

Alk stellt im Falle der gesättigten oder ungesättigten, geradkettigen oder verzweigten Alkylenkette Reste wie z.B. Methylen, Ethylen, Propylen, Butylen, 2-Methylpropylen, Pentylen, 1,1-Dimethyl-propylen, 2,3-Dimethyl-propylen, 2,2-Dimetyl-propylen, 2-Methyl-butylen, Hexylen, 2,3-Dimethyl-butylen, 2-Methyl-pentylen, 2-Butenylen, 2-Butinylen, insbesondere Methylen, Ethylen, Propylen, Butylen, 2-Methyl-propylen, Pentylen, Hexylen und 2-Butenylen dar.

Verbindungen der allgemeinen Formel I enthalten mindestens zwei asymmetrische Kohlenstoffatome, daher sind auch optisch aktive Verbindungen der allgemeinen Formel I Gegenstand der vorliegenden Anmeldung.

Verbindungen der allgemeinen Formel I werden nach an sich bekannten Verfahren aus Phosphonobernsteinsäureestern der allgemeinen Formel II, 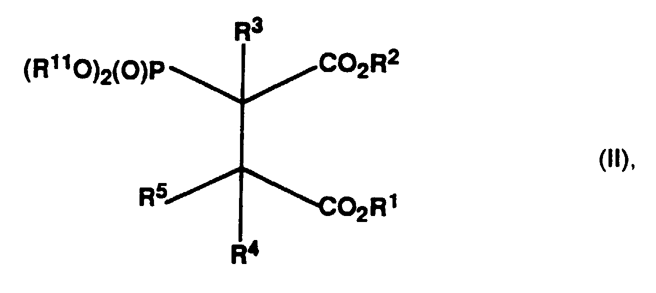 in der R¹, R², R³, R⁴, R⁵ die oben angegebenen Bedeutungen haben und R¹¹ einen Methyl-, Ethyl-, oder Benzylrest bedeutet, durch Verseifen hergestellt.

Verbindungen der allgemeinen Formel II werden nach an sich bekannten Verfahren hergestellt, vorzugsweise dadurch, daß man
a) Carbonsäurederivate der allgemeinen Formel III, 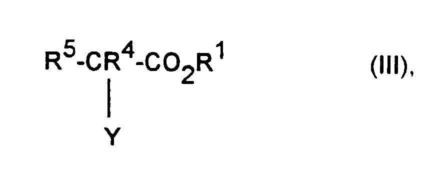 in der R⁴, R⁵, und R¹ die oben angegebenen Bedeutungen haben und Y eine Abgangsgruppe wie z.B. Hal oder O-SO₂-Z bedeuten, wobei Hal Chlorid, Bromid oder Jodid und Z Methyl, Phenyl, p-Methylphenyl oder p-Nitrophenyl sein sollen, mit einem Phosphonoessigsäureester der allgemeinen Formel IV, 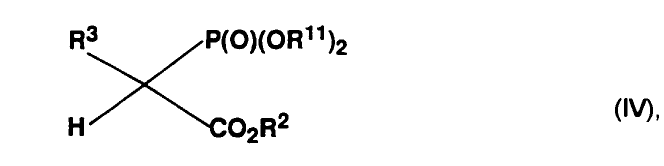 in der R², R³ und R¹¹ die oben angegebene Bedeutung besitzen, umsetzt, wobei für den Fall, daß R⁵ in Verbindungen der allgemeinen Formel III die freie Hydroxylgruppe bedeutet, diese in geschützter Form etwa als Acyloxy- Trialkylsilyloxy- oder Benzyloxygruppe vorliegen muß, und gegebenenfalls die enstandenen Ester teilweise oder vollständig zu den entsprechenden Säuren der allgemeinen Formel I verseift, oder, für den Fall, daß R⁴ Wasserstoff bedeutet,
b) Verbindungen der allgemeinen Formel V, 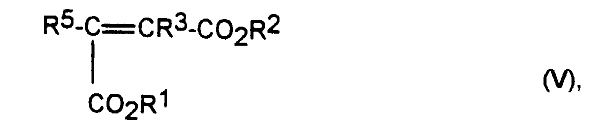 in der R⁵, R¹, R² und R³ die oben angegebenen Bedeutungen haben, mit einem Dialkylphosphit der allgemeinen Formel VI,

   H-P(O)(OR¹¹)₂ (VI),

   in der R¹¹ die oben angegebenen Bedeutungen hat, umsetzt und gegebenenfalls die entstandenen Ester teilweise oder vollständig zu den entsprechenden Säuren der allgemeinen Formel I verseift, oder, für den Fall, daß R³ = R⁴ = H ist,
c) eine Verbindung der allgemeinen Formel VII, 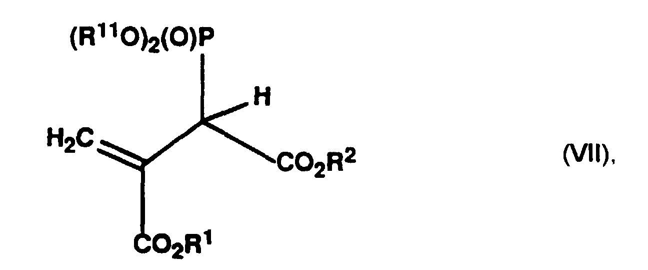 in der R¹, R² und R¹¹ die oben angegebene Bedeutungen besitzen, in an sich bekannter Weise mit einer Verbindung der allgemeinen Formel VIII,

   R⁵-M (VIII)

   in der R⁵ die oben genannten Bedeutungen besitzt, und M Wasserstoff oder ein Alkali- bzw. Erdalkalimetall bedeutet, zur Reaktion bringt und gegebenenfalls die enstandenen Ester teilweise oder vollständig zu den entsprechenden Säuren der allgemeinen Formel I verseift und gewünschtenfalls in pharmakologisch verträgliche Salze überführt,
d) eine Verbindung der allgemeinen Formel IX, 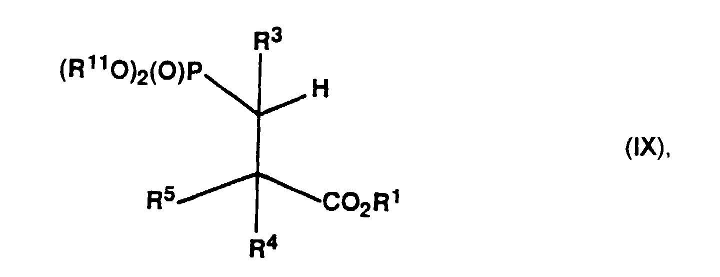 in der R¹, R³, R^{4,} R⁵ und R¹¹ die oben genannten Bedeutungen besitzen, nacheinander mit einer Base und einem Chlorkohlensäureester der allgemeinen Formel X, 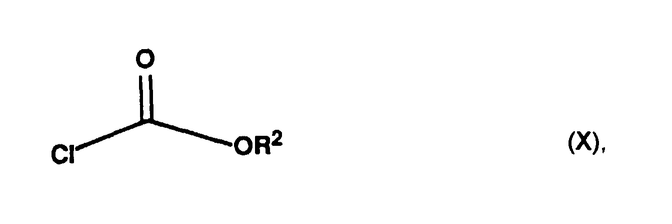 in der R² die oben genannten Bedeutungen besitzt, zur Reaktion bringt, oder
e) eine Verbindung der allgemeinen Formel Xl, 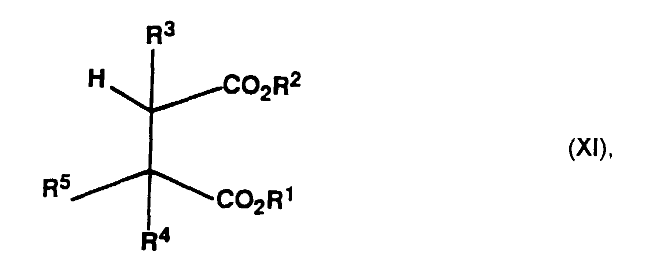 in der R¹, R², R³, R⁴ und R⁵ die oben genannten Bedeutungen besitzen, nacheinander mit einer Base und einer Verbindung der allgemeinen Formel XII,

   CIP(O)(OR¹¹)₂ (XII)

   in der R¹¹ die oben genannten Bedeutungen besitzt, umsetzt.
   Verbindungen der allgemeinen Formel I, bei denen R₃ Wasserstoff ist und die nach den Verfahren c) - e) hergestellt werden, können gegebenenfalls durch Behandlung mit einer Base und anschließend mit einer Verbindung der allgemeinen Formel XIII,

   R³-Y (XIII)

   in der R³ und Y die oben genannten Bedeutungen besitzt, in anderen Verbindungen der allgemeinen Formel I überführt werden.
f) Verbindungen der allgemeinen Formel I, bei denen R³ und R⁴ zusammen mit den Kohlenstoffatomen, an denen sie gebunden sind einen Ring bilden, lassen sich dadurch erhalten, daß man Verbindungen der allgemeinen Formel XIV oder XV, 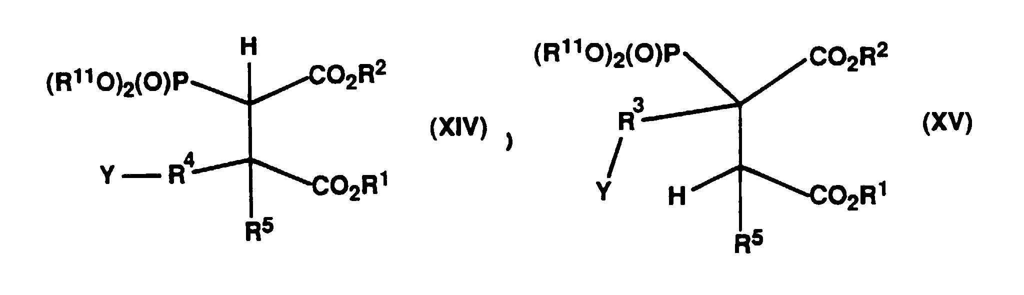 in der R¹, R², R³, R⁴, R⁵, R¹¹ und Y die oben genannten Bedeutungen besitzen, mit einer Base zur Reaktion bringt.

Verbindungen der allgemeinen Formel III werden so hergestellt, daß man für den Fall, daß Y = Hal bedeutet, eine Verbindung der allgemeinen Formel XVI,

R⁵-CHR⁴CO₂R¹ (XVI)

in der R¹, R⁴ und R⁵ die oben genannten Bedeutungen besitzen, nach literaturbekannten Verfahren halogeniert, oder für den Fall, daß Y in Formel III die O-SO₂-Z-Gruppe bedeutet, die Hydroxylgruppe einer Verbindung der allgemeinen Formel XVII, 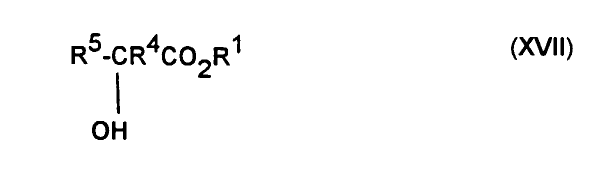 in der R¹, R⁴ und R⁵ die oben genannten Bedeutungen besitzen, in den entsprechenden Sulfonsäureester überführt.

Verbindungen der allgemeinen Formel IV sind teilweise käuflich erwerblich (Aldrich-Chemie GmbH u. Co.KG) und werden in Spezialfällen nach bekannten Verfahren durch Umsetzung eines Halogenessigsäurederivats der allgemeinen Formel XVIII, 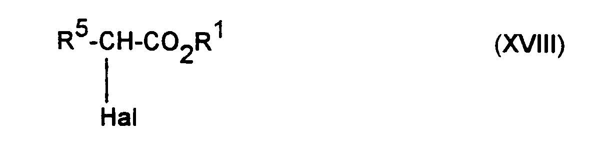 in der Hal, R¹ und R⁵ die oben angegebenen Bedeutungen besitzen, mit einem Triphosphit der allgemeinen Formel XIX,

P(OR¹¹)₃ (XIX)

in der R¹¹ die oben angegebenen Bedeutungen besitzt, hergestellt.

Verbindungen der allgemeinen Formel V werden dadurch hergestellt, daß man
eine Verbindung der allgemeinen Formel XX, 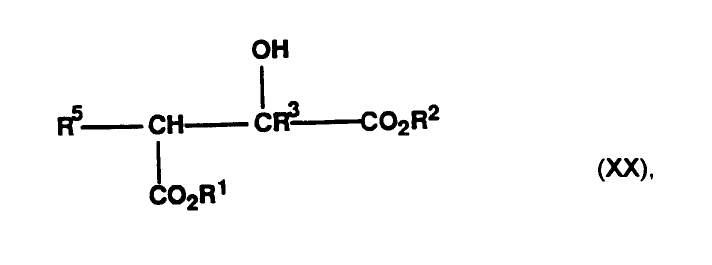 in der R¹, R², R³ und R⁵ die oben genannten Bedeutungen besitzen, nach bekannten Verfahren dehydratisiert.

Verbindungen der allgemeinen Formel VI sind käuflich erhältlich (Aldrich Co.).

Verbindungen der allgemeinen Formel VII werden in an sich bekannter Weise hergestellt, indem man eine Verbindung der allgemeinen Formel XXI, 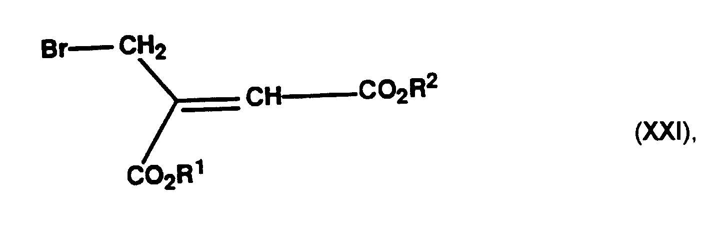 in der R¹ und R² die oben genannten Bedeutungen besitzen, mit einer Verbindung der allgemeinen Formel XIX umsetzt.

Verbindungen der allgemeinen Formel VIII werden für den Fall, daß M nicht Wasserstoff bedeutet, entsprechend den Literaturverfahren metalliert. Verbindungen der allgemeinen Formel IX werden nach bekannten Verfahren durch Alkylierung einer Verbindung der allgemeinen Formel XXII, 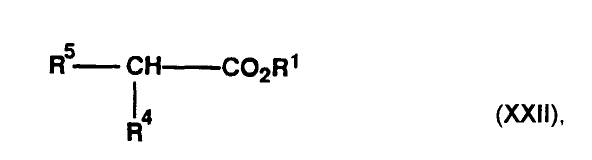 in der R¹, R⁴ und R⁵ die oben genannten Bedeutungen besitzen, mit einer Verbindung der allgemeinen Formel XXIII,

Hal-CH₂-P(O)(OR¹¹)₂ (XXIII)

in der Hal und R¹¹ die oben genannten Bedeutungen besitzen, erhalten.

Chlorkohlensäureester der allgemeinen Formel X sind käuflich erhältlich (Aldrich Co.).

Bernsteinsäurederivate der allgemeinen Formel Xl werden nach bekannten Verfahren durch Alkylierung einer Verbindung der allgemeinen Formel XXII mit einem Halogenessigsäureester der allgemeinen Formel XXIV,

Hal-CH₂-CO₂R² (XXIV)

in der Hal und R² die oben genannten Bedeutungen haben, hergestellt.

Verbindungen der allgemeinen Formel XII sind käuflich erhältlich (Aldrich Co.).

Verbindungen der allgemeinen Formel XIII sind, für den Fall, daß Y = Hal bedeutet, käuflich erhältlich; für den Fall, daß Y die O-SO₂-Z-Gruppe bedeutet, wird die Hydroxylgruppe käuflich erhältlicher Alkohole der allgemeinen Formel XXV,

R³-OH (XXV)

in der R³ die oben genannten Bedeutungen besitzt, in den entsprechenden Sulfonsäureester überführt.

Verbindungen der allgemeinen Formel XIV oder XV werden dadurch hergestellt, daß man die Hydroxylgruppe einer Verbindung der allgemeinen Formel XXVI oder XXVII, 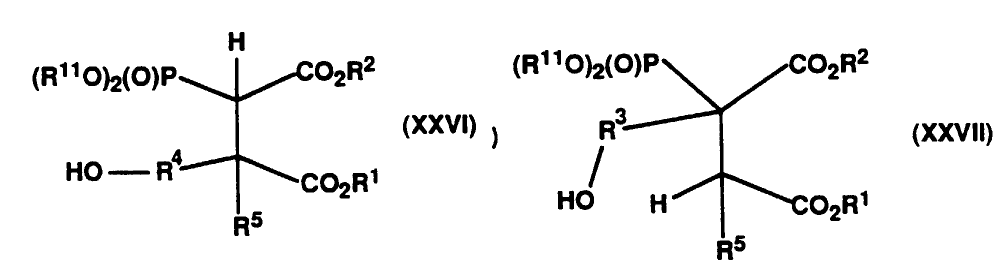 in der R¹, R², R³, R⁴, R⁵ und R¹¹ die oben genannten Bedeutungen besitzen, nach bekannten Verfahren in ein Halogen oder einen Sulfonsäureester überführt.
Carbonsäureester der allgemeinen Formel XVI werden nach bekannten Verfahren durch Alkylierung eines Carbonsäureesters der allgemeinen Formel XXVIII,

R⁵-CH₂-CO₂R¹ (XXVIII)

in der R¹ und R⁵ die oben genannten Bedeutungen besitzen, erhalten.

Verbindungen der allgemeinen Formel XVII lassen sich nach Literaturverfahren durch Oxydation der entsprechenden Verbindungen der allgemeinen Formel XVI erhalten.

Verbindungen der allgemeinen Formel XVIII werden nach literaturbekannten Methoden halogeniert.

Verbindungen der allgemeinen Formel XX werden in an sich bekannter Weise durch Reaktion einer Verbindung der allgemeinen Formel XXVIII mit einer Verbindung der allgemeinen Formel XXIX, 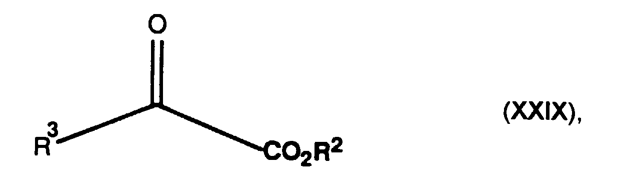 in der R² und R³ die oben genannten Bedeutungen besitzen, hergestellt.

Verbindungen der allgemeinen Formel XXI erhält man nach bekannten Methoden durch allylische Bromierung einer Verbindung der allgemeinen Formel XXX, 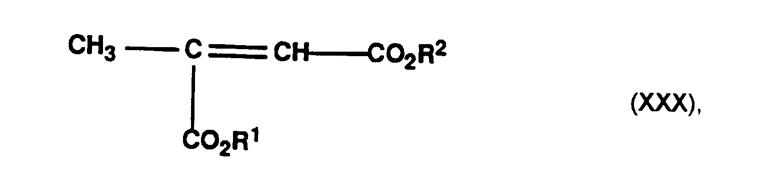 in der R¹ und R² die oben genannten Bedeutungen besitzen.

Verbindungen der allgemeinen Formel XXVI werden dadurch hergestellt, daß man eine Verbindung der allgemeinen Formel IV, in der R³ Wasserstoff bedeutet, mit einer Verbindung der allgemeinen Formel XXXI, 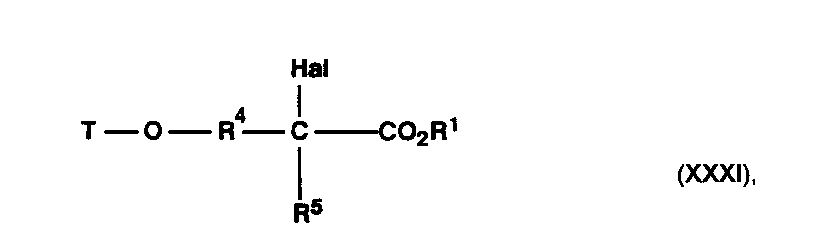 in der Hal, R¹, R⁴ und R⁵ die oben genannten Bedeutungen besitzen und T eine Hydroxy-Schutzgruppe, wie Benzyl-, Trimethylsilyl- oder tert.-Butyl-dimethylsilyl-Gruppe bedeutet, alkyliert und aus dem Reaktionsprodukt die Schutzgruppe T nach literaturbekannten Methoden entfernt.

Verbindungen der allgemeinen Formel XXVII lassen sich durch Alkylierung einer Verbindung der allgemeinen Formel XXXII, 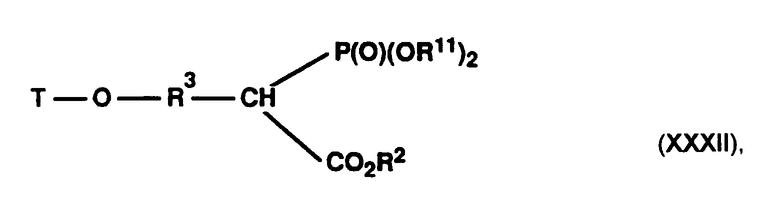 in der R², R³, R¹¹ und T die oben genannten Bedeutungen besitzen, mit einer Verbindung der allgemeinen Formel XVIII und anschließende Entfernung der Schutzgruppe T erhalten.

Verbindungen der allgemeinen Formel XXXI lassen sich nach bekannten Verfahren durch Halogenierung einer Verbindung der allgemeinen Formel XXXIII, 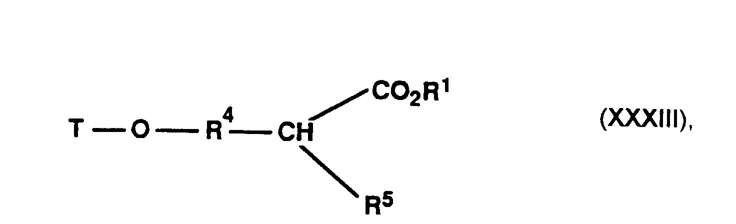 in der R¹ R⁴, R⁵ und T die oben genannten Bedeutungen besitzen, erhalten.

Verbindungen der allgemeinen Formel XXXII werden durch Alkylierung einer Verbindung der allgemeinen Formel IV, in der R³ Wasserstoff bedeutet, mit einer Verbindung der allgemeinen Formel XXXIV,

T-O-R³-Y (XXXIV)

in der R³, T und Y die oben genannten Bedeutungen besitzen, hergestellt.

Die Halogenierung einer Verbindung der allgemeinen Formel XVI oder der Formel XXXIII erfolgt durch ihre Umsetzung mit molekularem Halogen (Chlor, Brom, lod) vorzugsweise Brom ohne Lösungsmittel oder in einem inerten Lösungsmittel wie Methylenchlorid, Chloroform oder Tetrachlorkohlenstoff, vorzugsweise Tetrachlorkohlenstoff und unter Zusatz von rotem Phosphor, Phosphortrichlorid oder Phosphortribromid und bei einer Temperatur zwischen Raumtemperatur und 100°C, vorzugsweise bei 90°C (K. Stoh, Chem. Pharm. Bull. 34, 2078 (1986); H. J Ziegler, Synthesis 1969, 39)). Weiter lassen sich Verbindungen der allgemeinen Formel XVI dadurch halognieren, daß man sie in einem aprotischen Lösungsmittel wie Tetrahydrofuran und bei niedriger Temperatur, bevorzugt bei -78°C mit einem Lithiumamid wie Lithiumdiisopropylamid metalliert und anschließend die in a-Stellung metallierte Verbindungen der allgemeinen Formel XVI mit Brom, lod, Tetrachlorkohlenstoff oder Tetrabromkohlenstoff (M. Hesse, Helv. Chim. Acta 72, 847 (1989) R.T. Arnold, J. Org. Chem. 43, 3687 (1978)) bzw. mit N-Chlor- oder N-Bromsuccinimid (W. Oppolzer, Tetrahedron Lett. 26, 5037 (1985) umsetzt.

Die Überführung der Hydroxylgruppe einer Verbindung der allgemeinen Formel XVII, XXV, XXVI oder XXVII in einen Sulfonsäureester erfolgt nach üblichen Verfahren, wie z.B. durch die Kondensation mit einem Sulfonsäurechlorid, wie Methan-, Benzol, p-Toluol- oder p-Nitrobenzolsulfonsäurechlorid, vorzugsweise Methan- oder p-Toluolsulfonsäurechlorid, in einem inerten Lösungsmittel wie Methylenchlorid, Tetrahydrofuran oder Diethylether, vorzugsweise Methylenchlorid unter Verwendung einer Hilfsbase wie Tri- methyl- oder Triethylamin oder Pyridin, vorzugsweise Triethylamin und bei einer Temperatur zwischen 0°C und Raumtemperatur.

Die Umsetzung einer Verbindung der Formel III mit einer Verbindung der Formel IV, oder einer Verbindung der Formel IX mit einer Verbindung der Formel X, oder einer Verbindung der Formel Xl mit einer Verbindung der Formel XII, bzw. die Alkylierung einer Verbindung der Formel XXII mit einer Verbindung der Formel XXIII oder der Formel XXIV, oder die Alkylierung einer Verbindung der Formel IV mit einer Verbindung der Formel XXXI, oder einer Verbindung der Formel XXXII mit einer Verbindung der Formel XVIII, bzw. die Cyclisierung von Verbindungen der allgemeinen Formel XIV oder XV erfolgt in der Regel in einem aprotischen Lösungsmittel wie Toluol, Tetrahydrofuran, Diethylether oder Dimethylformamid, vorzugsweise Dimethylformamid, oder Tetrahydrofuran unter Verwandlung einer starken Base wie Kaliumhydrid, Natriumhydrid, Lithiumdiisopropylamid oder Lithiumhexamethyldisilylamid, vorzugsweise Natriumhydrid oder Lithiumdiisopropylamid und bei Temperaturen zwischen - 78°C und 90°C bevorzugt jedoch zwischen -10°C und Raumtemperatur.

Die Umsetzung einer Verbindung der allgemeinen Formel V mit einer Verbindung der allgemeinen Formel VI findet unter den Bedingungen der Michaeladdition, in einem Lösungsmittel wie Methanol, Ethanol, Toluol, Tetrahydrofuran, Diethylether, oder Dimethylformamid, vorzugsweise Methanol, Tetrahydrofuran oder Dimethylformamid ohne weitere Zusätze oder unter Verwendung einer Base wie Natrium- oder Kaliummethylat oder -ethylat, Natriumhydrid, Kaliumhydrid oder Lithiumdiisopropylamid, vorzugsweise Natriummethylat, Natriumhydrid oder Lithiumdiisopropylamid und bei Temperaturen zwischen -78°C und 90°C bevorzugt jedoch zwischen -10°C und Raumtemperatur statt.

Die Reaktion zwischen einer Verbindung der allgemeinen Formel VII mit einer Verbindung der allgemeinen Formel VIII führt man in der Regel unter den Bedingungen der Michaeladdition in einem Lösungsmittel wie Methanol, Ethanol, Toluol, Tetrahydrofuran, Diethylether oder Dimethylformamid, vorzugsweise Methanol, Tetrahydrofuran oder Dimethylformamid ohne weitere Zusätze oder unter Verwendung einer Base wie Natriumhydrid, Kaliumhydrid, Lithiumdiisopropylamid, Butyllithium, Ethylmagnesiumbromid, und gegebenenfalls Kupfersalz, wie Kupferchlorid- oder bromid zur Bildung des entsprechenden Cuprates einer Verbindung der allgemeinen Formel VIII (vgl. G.H. Posner, Tetrahedron Letters 37, 3215 (1977)), und bei Temperaturen zwischen -78°C und 90°C bevorzugt zwischen -78°C und Raumtemperatur durch.

Die Reaktion zwischen einer Verbindung der allgemeinen Formel XVIII mit einer Verbindung der allgemeinen Formel XIX erfolgt in der Regel ohne Lösungsmittel bei Temperaturen zwischen Raumtemperatur und 150°C, vorzugsweise bei 130°C mit einer Reaktionszeit zwischen 30min und 30 Stunden, vorzugsweise 18 Stunden.

Die Dehydratisierung einer Verbindung der allgemeinen Formel XX findet gewöhnlich in einem Lösungsmittel wie Benzol, Toluol, Xylol, Chloroform oder Methylenchlorid, vorzugsweise Toluol oder Methylenchlorid unter Zusatz eines Dehydratisierungsmittels wie Schwefelsäure, Phosphorsäure, p-Toluolsulfonsäure, vorzugsweise p-Toluolsulfonsäure und bei einer Temperatur zwischen Raumtemperatur und Rückflußtemperatur des verwendeten Lösungsmittels, vorzugsweise bei 100°C, statt.

Die Reaktion einer Verbindung XIX mit einer Verbindung XXI erfolgt in der Regel ohne Lösungsmittel bei Temperaturen zwischen 50°C und 180°C vorzugsweise bei 150°C.

Die Kondensation eines Carbonsäureesters der allgemeinen Formel XXVIII mit einem Keton der Formel XXIX führt man gewöhnlich in einem Lösungsmittel wie Methanol, Ethanol, Tetrahydrofuran, Diethylester oder Dimethylformamid, vorzugsweise Methanol oder Tetrahydrofuran in Gegenwart eines basischen Kondensationsmittels wie Natriummethylat oder -ethylat. Kalium-tert.-butylat, Natriumhydrid oder Lithiumdiisopropylamid, vorzugsweise Natriummethylat, Kalium-tert.-butylat oder Lithiumdiisopropylamid und bei Temperaturen zwischen -78°C und 60°C, vorzugsweise zwischen -78°C und Raumtemperatur durch.

Zur allylischen Bromierung von 2-Methylfumar- oder maleinsäure und ihren Derivaten siehe J. Org. Chem 34, 1228 (1969). Die Oxidation einer Verbindung der allgemeinen Formel XVI zu einer Verbindung der allgemeinen Formel XVII führt man in der Regel in einem Lösungsmittel wie Tetrahydrofuran durch Zugabe einer Base wie Lithiumdiisopropylamid oder Lithium-N-Isopropyl-N-Cyclohexylamid unter Verwendung eines Oxidationsmittels wie einem Oxaziridin-Derivat, Molibdänperoxid oder Luftsauerstoff und bei Temperaturen zwischen -78°C und Raumtemperatur, vorzugsweise bei 50°C durch (C. Tamm. Tetrahedron Lett. 26, 203 (1985); F. A. Davis I. Org. Chem. 51, 2402 (1986); C. Winotai Synth. Commun. 18, 2141 (1988)).

Die Hydrolyse einer Phosphonsäureester-Gruppe in Verbindung der allgemeinen Formel II zu der entsprechenden freien Phosphonsäure-Gruppe erfolgt in der Regel ohne Lösungsmittel oder in einem inerten Lösungsmittel wie Methylenchlorid durch ein Trimethylsilylhalogenid, wie Trimethylsilylbromid oder -jodid und bei einer Temperatur zwischen -50°C und Raumtemperatur, vorzugsweise bei 0°C.

Die Verseifung einer Carbonsäureester-Gruppe in Verbindungen der allgemeinen Formel I oder II führt man nach üblichen Verfahren durch, indem man einen Carbonsäureester der allgemeinen Formel I oder II in Wasser oder in Gemischen aus Wasser, Tetrahydrofuran, Dioxan, Methanol oder Ethanol, vorzugsweise in einem Wasser/Tetrahydrofurangemisch mit einem Hydroxid wie Natrium-, Kalium- oder Lithiumhydroxid, vorzugsweise Natrium- oder Lithiumhydroxid und bei Temperaturen zwischen Raumtemperatur und 80°C, vorzugsweise bei Raumtemperatur, behandelt.

Die Schutzgruppe einer Hydroxylgruppe in Verbindungen der allgemeinen Formel III oder XXXI läßt sich dadurch entfernen, daß man nach üblichen Verfahren eine Verbindung der allgemeinen Formel III oder XXXI mit wäßrigen Mineralsäuren bzw. -basen, wie Salzsäure oder Schwefelsäure bzw. Natron- oder Kalilauge behandelt, oder mit einem Fluorid, wie wäßrige Fluorwasserstoffsäure oder Tetrabutylammoniumfluorid umsetzt, oder diese einer katalytischen Hydrierung, wie z. B. mit Palladium/Kohle/Wasserstoff unterwirft.

Zu reinen Enantiomeren der Verbindungen der Formel I kommt man entweder durch Racematspaltung (über Salzbildung mit optisch aktiven Säuren oder Basen), oder indem man in die Synthese optisch aktive Ausgangsstoffe einsetzt.

Phosphon- und Carbonsäureester-Gruppen in Verbindungen der allgemeinen Formel I oder II lassen sich weiterhin durch Kochen mit Chlor- oder Bromwasserstoffsäure verseifen. Liegen in Verbindungen der allgemeinen Formel I oder II Benzylester vor, so lassen sie sich hydrogenolytisch in die entsprechenden freien Phosphon- bzw. Carbonsäuren überführen.

Als pharmakologisch verträgliche Salze werden vor allem Mono- bzw. Di-alkali- oder Ammoniumsalze verwendet, die man in üblicher Weise z. B. durch Titration der Verbindungen mit anorganischen oder organischen Basen wie z.B. Natrium-oder Kaliumhydrogencarbonat, Natronlauge, Kalilauge, wässrigem Ammoniak oder Aminen wie z.B. Trimethyl- oder Triethylamin herstellt. Die Salze werden in der Regel durch Umfällen aus Wasser/Aceton gereinigt.

Die erfindungsgemäßen neuen Substanzen der Formel I und ihre Salze können in flüssiger oder fester Form enteral oder parenteral appliziert werden. Hierbei kommen alle üblichen Applikationsformen infrage, beispielsweise Tabletten, Kapseln, Dragees, Sirupe, Lösungen, Suspensionen etc.. Als Injektionsmedium kommt vorzugsweise Wasser zu Anwendung, welches die bei Injektionslösungen üblichen Zusätze wie Stabilisierungsmittel, Lösungsvermittler und Puffer enthält.

Derartige Zusätze sind z.B. Tartrat- und Citrat-Puffer, Ethanol, Komplexbildner (wie Ethylendiamintetraessigsäure und deren nichttoxische Salze), hochmolekulare Polymere (wie flüssiges Polyethylenoxid) zur Viskositätsregelung.

Flüssige Trägerstoffe für Injektionslösungen müssen steril sein und werden vorzugsweise in Ampullen abgefüllt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole); für orale Applikation geeignete Zubereitungen können gewünschtenfalls Geschmacks- und Süßstoffe enthalten.

Die Dosierung kann von verschiedenen Faktoren, wie Applikationsweise, Spezies, Alter und/oder individuellem Zustand abhängen. Die tägliche zu verabreichenden Dosen liegen bei etwa 10-1000 mg/Mensch, vorzugsweise bei 100-500 mg/Mensch und können auf einmal oder mehrere Male verteilt eingenommen werden.

Bevorzugt im Sinne der vorliegenden Erfindung sind außer den in den Beispielen genannten Verbindungen und durch Kombination aller in den Ansprüchen genannten Bedeutungen der Substituenten ableitbaren Verbindungen die folgenden Bernsteinsäurederivate, sowie deren Natrium- und Kaliumsalze, Methyl-, Ethyl- oder Benzylester:

### Bevorzugte Verbindungen (BV):

1) 2-Phosphono-2-methyl-3-butyl-bernsteinsäure
2) 2-Phosphone-3-butyl-bernsteinsäure, Smp. 157-9°C (Zers.)
3) 2-Phosphono-3-pentyl-bernsteinsäure
4) 2-Phosphono-3-(3-methyl-1-butyl)-bernsteinsäure, Smp. 151-2°C (Zers.)
5) 2-Phosphono-3-methyl-3-butyl-bernsteinsäure
6) 2-Phosphono-3-(2-propen-1-yl)-bernsteinsäure
7) 2-Phosphono-3-(2-methyl-2-propen-1-yl)-bernsteinsäure
8) 2-Phosphono-3-cyclohexyl-bernsteinsäure
9) 2-Phosphono-3-(2-cyclohexen-1-yl)-bernsteinsäure
10) 2-Phosphono-3-(phenylmethyl)-bernsteinsäure, Natriumsalz: ¹H-NMR (D₂O): 3.35 (dd, 1H), 3.25-3.0 (m, 3H), 2.92-2.80 (m, 1H); ³¹P-NMR (D₂O): 18.01 (s), 16.99 (s).
11) 2-Phosphono-3-(4-hydroxycyclohexyl)-bernsteinsäure
12) 2-Phosphono-3-(4-aminocyclohexyl)-bernsteinsäure
13) 2-Phosphono-3-(4-dimethylaminocyclohexyl)-bernsteinsäure
14) 2-Phosphono-3-(4-hydroxy-cyclohex-2-en-1-yl)-bernsteinsäure
15) 2-Phosphono-3-(2-hydroxycyclopentyl)-bernsteinsäure
16) 2-Phosphono-3-(4-isopropylcyclohexyl)-bernsteinsäure
17) 2-Phosphono-3-cylcoheptyl-bernsteinsäure
18) 2-Phosphono-3-(2-phenylethyl)-bernsteinsäure
19) 2-Phosphono-3-(3-phenylpropyl)-bernsteinsäure
20) 2-Phosphono-3-(4-methoxyphenylmethyl)-bernsteinsäure
21) 2-Phosphono-3-(4-hydroxyphenylmethyl)-bernsteinsäure
22) 2-Phosphono-3-(4-dimethylaminophenylmethyl)-bernsteinsäure
23) 2-Phosphono-3-(4-aminophenylmethyl)-bernsteinsäure
24) 2-Phosphono-3-(4-methylphenylmethyl)-bernsteinsäure
25) 2-Phosphono-3-(3-chlorphenylmethyl)-bernsteinsäure
26) 2-Phosphono-3-(2-methoxyphenylmethyl)-bernsteinsäure
27) 2-Phosphono-3-(4-aminocarbonylphenylmethyl)-bernsteinsäure
28) 2-Phosphono-3-(4-amidinophenylmethyl)-bernsteinsäure
29) 2-Phosphono-3-(4-Cyanophenylmethyl)-bernsteinsäure
30) 2-Phosphono-3-(3,4-dimethoxyphenylmethyl)-bernsteinsäure
31) 2-Phosphono-3-(2-(3,4-dimethoxyphenyl)-ethyl)-bernsteinsäure
32) 2-Phosphono-3-(2-(4-aminophenyl)ethyl)-bernsteinsäure
33) 2-Phosphono-3-(1-naphthyl)-bernsteinsäure
34) 2-Phosphono-3-(2-naphthyl)-bernsteinsäure, Smp.180-2°C (Zers.)
35) 2-Phosphono-3-(4-phenylphenyl)-bernsteinsäure
36) 2-Phosphono-3-(4-(4-amidinophenyl)phenyl)-bernsteinsäure
37) 2-Phosphono-2-amino-3-methyl-bernsteinsäure
38) 2-Phosphono-2-hydroxy-3-methyl-bernsteinsäure
39) 2-Phosphono-3-amino-3-methyl-bernsteinsäure
40) 2-Phosphono-3-hydroxy-3-methyl-bernsteinsäure
41) 2-Phosphono-2-amino-3-ethyl-bernsteinsäure
42) 2-Phosphono-2-methoxy-3-ethyl-bernsteinsäure
43) 2-Phosphono-3-amino-3-ethyl-bernsteinsäure
44) 2-Phosphono-3-methoxy-3-ethyl-bernsteinsäure
45) 2-Phosphono-2-amino-3-phenyl-bernsteinsäure
46) 2-Phosphono-3-amino-3-phenyl-bernsteinsäure
47) 2-Phosphono-2-methoxy-3-phenyl-bernsteinsäure
48) 2-Phosphono-2-amino-3-phenylmethyl-bernsteinsäure
49) 2-Phosphono-3-methoxy-3-phenylmethyl-bernsteinsäure
50) 2,3-Dimethoxy-2-phosphono-bernsteinsäure
51) 2-Phosphono-2-methoxy-3-(4-phenyl)phenyl-bernsteinsäure
52) 2-Phosphono-3-(2-cyclohexyl)ethyl-bernsteinsäure
53) 2-Phosphono-3-ethyloxy-bernsteinsäure
54) 2-Phosphono-3-butyloxy-bernsteinsäure
55) 2-Phosphono-3-cyclohexyloxy-bernsteinsäure
56) 2-Phosphono-3-phenyloxy-bernsteinsäure
57) 2-Phosphono-3-(4-hydroxyphenyloxy)-bernsteinsäure
58) 2-Phosphono-3-(4-dimethylaminophenyloxy)-bernsteinsäure
59) 2-Phosphono-3-(1-naphthyloxy)-bernsteinsäure
60) 2-Phosphono-3-(4-(1-pyrrolidinopropyl)phenyloxy)-bernsteinsäure
61) 2-Phosphono-3-(4-(1-pyrrolidinopropyloxy)phenyloxy)-bernsteinsäure
62) 2-Phosphono-3-(4-phenyl)phenyloxy)-bernsteinsäure
63) 2-Phosphono-3-(4-(4-amidinophenyl)phenyloxy)-bernsteinsäure
64) 2-Phosphono-3-hydroxymethyl-bernsteinsäure
65) 2-Phosphono-3-methoxymethyl-bernsteinsäure
66) 2-Phosphono-3-propoxymethyl-bernsteinsäure
67) 2-Phosphono-3-cyclohexyloxymethyl-bernsteinsäure
68) 2-Phosphono-3-phenoxymethyl-bernsteinsäure
69) 2-Phosphono-3-(4-hydroxyphenyl)oxymethyl-bernsteinsäure
70) 2-Phosphono-3-(4-dimethylaminophenyl)oxymethyl-bernsteinsäure
71) 2-Phosphono-3-( 1 -naphthyloxymethyl)-bernsteinsäure
72) 2-Phosphono-3-(4-(phenyl)phenyl)oxymethyl-bernsteinsäure
73) 2-Phosphono-3-(2-hydroxyethyl)-bernsteinsäure
74) 2-Phosphono-3-(3-methoxypropyl)-bernsteinsäure
75) 2-Phosphono-3-(3-phenoxypropyl)-bernsteinsäure
76) 2-Phosphono-3-(4-(4-methoxyphenylmethyloxy)butyl-bernsteinsäure
77) 2-Phosphono-3-methylthio-bernsteinsäure
78) 2-Phosphono-3-propylthio-bernsteinsäure, Smp. 154-5°C
79) 2-Phosphono-3-propyl-thiomethyl-bernsteinsäure, Smp. 80°C (Zers.)
80) 2-Phosphono-3-phenylthio-bernsteinsäure, Smp. 149-50°C
81) 2-Phosphono-3-methylthiomethyl-bernsteinsäure
82) 2-Phosphono-3-ethylthio-bernsteinsäure, Smp. 160-2°C
83) 2-Phosphono-3-ethylthiomethyl-bernsteinsäure, Smp. 176-7°C
84) 2-Phosphono-3-phenylthiomethyl-bernsteinsäure, Smp. 171-2°C
85) 2-Phosphono-3-(4-(4-methoxyphenyl)thiobutyl)-bernsteinsäure
86) 2-Phosphono-3-(2-piperidino)-bernsteinsäure
87) 2-Phosphono-3-(2-tetrahydropyranyl)-bernsteinsäure
88) 2-Phosphono-3-(2-(4-methoxy)piperidino)-bernsteinsäure
89) 2-Phosphono-3-(3-phenoxyethyl)-bernsteinsäure, Natriumsalz: ¹H-NMR (D₂O): 7.40 (dd, 2H), 7.05 (m, 3H), 4.20 (m, 2H), 3.50-3.15 (m, 2H), 2.65 (m, 1H), 2.22 (m, 1H); ³¹P-NMR (D₂O): 15.17 (s), 14.53 (s)
90) 2-Phosphono-3-((2-piperidino)methyl)-bernsteinsäure
91) 2-Phosphono-3-((2-tetrahydropyranyl)methyl)-bernsteinsäure
92) 2-Phosphono-3-(2-(2-piperidino)ethyl)-bernsteinsäure
93) 2-Phosphono-3-(2-(4-hydroxy)pyrrolidino)-bernsteinsäure
94) 2-Phosphono-3-(2-tetrahydrofuranyl)-bernsteinsäure
95) 2-Phosphono-3-(2-tetrahydrothiophenyl)-bernsteinsäure
96) 2-Phosphono-3-((2-tetrahydrofuranyl)methyl)-bernsteinsäure
97) 2-Phosphono-3-((2-tetrahydrothiophenyl)methyl)-bernsteinsäure
98) 2-Phosphono-3-(3-(2-pyrrolidino)propyl)-bernsteinsäure
99) 1 -Phosphono-1,2cyclohexan-dicarbonsäure
100) 1-Phosphono-1,2-cyclopentan-dicarbonsäure
101) 3-Phosphono-2,3-piperidin-dicarbonsäure
102) 3-Phosphono-2,3-pyrrolidin-dicarbonsäure
103) 3-Phosphono-2,3-tetrahydropyran-dicarbonsäure
104) 3-Phosphono-2,3-tetrahydrofuran-dicarbonsäure
105) 2-Phosphono-2,3-morpholin-dicarbonsäure
106) 2-Phosphono-3-(4-hydroxyphenylmethyl)-bernsteinsäure: ¹H-NMR (D₂O): 7.26 (d, 1H), 7.22 (d, 1H), 6.90 (d, 2H), 3.12-2.98 (m, 2H), 2.93-2.80 (m, 2H); ³¹P-NMR (D₂O): 17.82 (s), 15.93 (s).

Die nachfolgenden Beispiele zeigen einige der Verfahrensvarianten, die zur Synthese der erfindungsgemäßen Verbindungen verwendet werden können. Sie sollten jedoch nicht eine Einschränkung des Erfindungsgegenstandes darstellen. Die Struktur der Verbindungen wurde durch ¹H-, ³¹p- und gegebenenfalls durch ¹³C-NMR-Spektroskopie gesichert. Die Reinheit der Substanzen wurde mittels C, H, N, P, gegebenenfalls Na-Analyse sowie dünnschichtchromtagraphisch bzw. durch Dünnschichtelektrophorese (Cellulose, Oxalat-Puffer von pH = 4.0) bestimmt.

### Beispiel 1

### 2-Phosphono-3-Cyclohexylmethyl-bernsteinsäure

a) Zu der auf 80°C erhitzten Mischung aus 6.8 ml (40 mmol) 3-Cyclohexylpropionsäure und 0.8 g (26 mmol) rotem Phosphor in 40 ml Tetrachlorkohlenstoff tropft man innerhalb zwei Stunden 8.2 ml (160 mmol) Brom. Danach erhitzt man die Reaktionsmischung noch 15 h am Rückfluß, kühlt sie dann ab und zieht das Lösungsmittel am Rotationsverdampfer ab. Der zurückgebliebene Rückstand wird unter Eiskühlung mit 50 ml Ethanol versetzt und die erhaltene Lösung eine Stunde am Rückfluß erhitzt. Anschließend wird die Reaktionslösung zur Trockne eingedampft, der Rückstand in 30 ml gesätt. Natriumhydrogencarbonat-Lösung aufgenommen und die so erhaltene wäßrige Lösung dreimal mit je 50 ml Ether extrahiert. Die vereinigten Etherphasen werden mit 10 ml gesätt. Natriumthiosulfat-Lösung geschüttelt und über Natriumsulfat getrocknet. Nach Abziehen des Ethers erhält man 10 g 2-Brom-3-cyclohexylpropionsäureethylester als gelbes Öl. ¹H-NMR (CDCl₃): δ = 4.33 ppm (t, 1H); 4.16 (q, 2H); 1.85 (t, 2H); 1.73-1.28 (m, 6H); 1.23 (t, 3H); 1.22-0.74 (m, 5H).
b) Die Lösung von 4 ml (20 mmol) Phosphonoessigsäuretriethylester in 30 ml absolutem Dimethylformamid (abs. DMF) wird bei Raumtemperatur mit 0.48 g (20 mmol) Natriumhydrid versetzt und solange gerührt, bis die Wasserstoffentwicklung beendet ist (30 min). Zu dieser Lösung tropft man dann 5.26 g (20 mmol) des oben hergestellten 2-Brom-3-cyclohexyl-propionsäureethyl-esters in 20 ml abs. DMF und läßt anschließend die Reaktionsmischung 8 h bei Raumtemperatur rühren. Danach wird das DMF am Rotationsverdampfer abgezogen, der Rückstand in 20 ml gesätt. Ammoniumchlorid-Lösung aufgenommen und die entstandene wäßrige Lösung dreimal mit je 50 ml Essigsäureethylester extrahiert. Nach dem Trocknen der vereinigten organischen Phasen über Natriumsulfat und Abziehen des Lösungsmittels wird das Rohprodukt an Kieselgel säulenchromatographisch gereinigt (Laufmittel: Essigsäureethylester/Isohexan = 2/1). Man erhält 3.5 g 2-Phosphono-3-cyclohexylmethyl-bernsteinsäuretetraethyles ter als farbloses Öl. ¹H-NMR (CDCl₃): δ = 4 ppm (m, 8H); 3.26 (q, 1H); 3.15-2.97 (m, 1H); 1.99-1.4 (m, 6H); 1.33-1.0 (m, 17H); 0.97-0.68 (m, 2H); (das Produkt liegt als Diastereomeren-Gemisch im Verhältnis 1:1 vor). ³¹P-NMR (CDCl₃): δ = 19.18 ppm; δ = 18.61 ppm.
c) 2.5 g (6.2 mmol) 2-Phosphono-3-cyclohexylmethyl-bernsteinsäuretetraethyles ter werden in 50 ml 6 N Salzsäure suspendiert. Die Suspension wird dann 24 h am Rückfluß erhitzt und anschließend die abgekühlte wäßrige Lösung zweimal mit je 50 ml Essigsäureethylester ausgeschüttelt. Nach Eindampfen der wäßrigen Phase erhält man 1.3 g 2-Phosphono-3-cyclohexylmethyl-bernsteinsäure. Diese wird in 10 ml Wasser gelöst und die so erhaltene saure Lösung mittels 1 N Natronlauge auf pH 7 (pH-Elektrode) eingestellt. Nach Abziehen des Wassers wird der Rückstand in Diethylether suspendiert und die Suspension abfiltriert. Man erhält so 1.2 g 2-Phosphono-3-cyclohexyl- methyl-bernsteinsäure-Trinatriumsalz. Fp. > 250°C. C,H-Analyse C₁₁H₁₆Na₃O₇P x 2.5 H₂O Ber. C 32.60, H 5.22; Gef. C 32.63, H 5.25.

Analog zum Beispiel 1 wurde, ausgehend von 3-Phenylpropionsäure, 2-Phosphono-3-phenylmethyl-bernsteinsäure hergestellt. Die Verbindung liegt als Diastereomerengemisch im Verhältnis 1/1 vor. ¹H-NMR (D₂O); δ = 7.4 ppm (m, 5H); 3.5- 2.85 (m, 2H); ³¹P-NMR (D₂O): δ = 15.25 ppm (s).

### Beispiel 2

### 2-Phosphono-3-(3,4-dimethoxyphenyl)methyl-bernsteinsäure

a) 85.68 ml 1.6 N Butyllithium-Lösung in Hexan (0.136 mol) wurden bei -10°C unter Stickstoff mit 19.27 ml (0.136 mol) Diisopropylamin versetzt. Danach wurde die weiße Suspension 30 min bei -10°C gerührt, dann mit 200 ml absolutem Tetrahydrofuran (abs. THF) versetzt und die erhaltene Lithiumdiisopropylamid-Lösung auf -78°C abgekühlt. Anschließend gab man schnell die Lösung von 25.6 ml (0.2 mol) frisch destillierten Chlortrimethylsilan in 20 ml abs. THF zu, tropfte dann innerhalb von 10 min eine Lösung von 24.5 g (0.109 mol) 3-(3,4-Dimethoxyphenyl)-propionsäure-methylester in 50 ml abs. THF und ließ danach die Reaktionsmischung eine Stunde bei -78°C rühren. Anschließend fügte man 22.6 g (0.127 mol) N-Bromsuccinimid hinzu, ließ die Reaktionsmischung auf Raumtemperatur kommen (1 h) und rührte sie dann 8 h weiter. Nach Abfiltrieren des entstandenen Niederschlags wurde die Mutterlauge im Vakuum eingeengt, der Rückstand in 100 ml Diethylether aufgenommen, die Etherlösung mit gesättigter Natriumthiosulfat-Lösung gewaschen und über Natriumsulfat getrocknet. Nach Abziehen des Ethers erhielt man 33.8 g 2-Brom-3-(3,4-dimethoxyphenyl)-propionsäuremethylester als leicht braunes Öl. ¹H-NMR (CDCl₃): δ = 6.75 (m, 3H); 4.35 (dd,1H); 3.85 (s, 3H); 3.83 (s, 3H); 3.7 (s, 3H); 3.4 (dd, 1H); 3.15 (dd, 1H).
b) Die Alkylierung von Phosphonoessigsäuretriethylester mit 2-Brom-3-(3,4-dimethoxyphenyl)-propionsäuremethylester, die analog zum Beispiel 1 b) erfolgte, ergab den 2-Diethylphosphono-3-(3,4-dimethoxyphenyl)methyl-bernstein säuremethyl-ethylester. Dieser liegt als Diastereomeren-Gemisch im Verhältnis 1/2 vor. ¹H-NMR (CDCl₃): δ = 6.73 ppm (d, 1H); 6.68 (s, 1H); 6.67 (d, 1H); 4.14 (m, 6H); 3.78 (zwei s, 2 x 3H); 3.47 (s, 3H); 3.39-3.18 (m, 3H); 2.97-2.70 (m, 1H); 1,22 (m, 9H); ³¹p-NMR (CDCl₃): δ = 18.34 ppm (S); δ = 18.17 ppm (s).
c) Die Verseifung der Estergruppen in 2-Diethylphosphono-3-(3,4-dimethoxy-phenyl)methyl-bernstei nsäure-methyl-diethylester wurde analog zum Beispiel 1 c) durchgeführt und ergab die freie 2-Phosphono-3-(3,4-dimethoxyphenyl)-methyl-bernsteinsäure Fp. 195°C (Zers.).

### Beispiel 3

### 2-Phosphono-3-(4-hydroxybutyl)-bernsteinsäure

a) Die Mischung von 10.6 ml (0.1 mol) e-Caprolacton und 1.97 g (0.015 mol) Kaliumcarbonat in 50 ml Methanol wird eine Stunde bei Raumtemperatur gerührt, dann das Kaliumcarbonat abfiltriert und die Mutterlauge i. V. zur Trockne eingedampft. Der Rückstand wird in 20 ml gesätt. Ammoniumchlorid-Lösung aufgenommen und die Lösung dreimal mit je 50 ml Dietyhlether extrahiert. Nach Trocknen der vereinigten organischen Phasen über Natriumsulfat und Abziehen des Lösungsmittels erhält man 12.2 g 6-Hydroxycapronsäuremethylester, der ohne zusätzliche Reinigung weiter umgesetzt wird.
b) Die Lösung von 5.8 g (40 mmol) 6-Hydroxycapronsäuremethylester und 8.2 g (120 mmol) Imidazol in 50 ml trockenem Dimethylformamid (DMF) wurde mit 6.8 g (45 mmol) tert.-Butyldimethylsilylchlorid versetzt. Anschließend ließ man die Reaktionsmischung 4 h bei Raumtemperatur rühren, zog dann das DMF i. V. ab, nahm den Rückstand in 500 ml Wasser auf und schüttelte die wäßrige Lösung dreimal mit je 100 ml Diethylether. Nach Trocknen der vereinigten Etherphasen über Natriumsulfat und Abziehen des Lösungsmittels wurde das Rohprodukt an Kieselgel säulenchromatographisch gereinigt. (Laufmittel: Essigsäureethylester/Isohexan = 1/10). Man erhielt so 4.1 g 6-tert.-Butyl- dimethylsilyloxycapronsäuremethylester. ¹H-NMR (CDCl₃): δ = 3.61 ppm (s, 3H); 3.55 (t, 2H); 2.26 (t, 2H); 1.60 (m, 2H); 1.49 (m, 2H); 1.30 (m, 2H); 0.85 (s, 9H); 0.001 (s, 6H).
c) Die Tieftemperatur-Bromierung von 6-tert.-butyldimethylsilyloxycapron-säuremethylester, die in Analogie zum Beispiel 2 a) erfolgte, lieferte den 2-Brom-6-tert. butyldimethylsilyloxycapronsäuremethylester. ¹H-NMR (CDCl₃): δ = 4.19 ppm (dd, 1H); 3.70 (s, 3H); 3.52 (t, 2H); 2.0 (m, 2H); 1.52-1.40 (m, 4H); 0.83 (s, 9H); 0.001 (s. 6H).
d) Die Alkylierung von Phosphonoessigsäuretriethylester mit 2-Brom-6-tert.-butyldimethylsilyloxy-capronsäuremethyleste r, die analog zum Beispiel 1 b) durchgeführt wurde, ergab den 2-Phosphono-3-(4-tert.-butyldimethylsilyl-oxybutyl)-bernst einsäure-methyl-triethylester als hellgelbes Öl. ¹H-NMR (DMSO-d⁶) (Diastereomeren-Gemisch 1/1): 4.05 (m, 6H); 3.55 (s, 3H); 3.50 (m, 2H); 3.15 (m,1H); 2.85 (m, 1H); 1.40 (m, 2H); 1.15 (m, 4H+9H); 0.85 (s, 9H); 0.001 (s, 6H); ³¹p-NMR (DMSO-d⁶): δ = 24.79 ppm (s); δ = 24.18 ppm (s).
e) Die Lösung von 1.4 g (2.9 mmol) 2-Phosphono-3-(4-tert.butyldimethylsilyl-oxybutyl)-bernst einsäure-methyl-triethylester in 5 ml Acetonitril wird mit 3 ml 2proz. (in Acetonitril) Fluorwasserstoffsäure versetzt. Anschließend wird die Reaktionsmischung 2 h bei Raumtemperatur gerührt, danach das Lösungsmittel i. V. abgezogen, der Rückstand in 10 ml gesätt. Natriumhydrogencarbonat-Lösung aufgenommen und die wäßrige Lösung dreimal mit je 20 ml Essigesäureethylester extrahiert. Nach Trocknen der vereinigten organischen Phasen über Natriumsulfat und Abziehen des Lösungsmittels erhält man 0.6 g 2-Phosphono-3-(4-hydroxybutyl)-bernsteinsäure-methyl-triet hylester als farbloses zähes Öl. ¹H-NMR (CDCl₃) (Diastereomeren-Gemisch 1/1): δ = 4.05 ppm (m, 6H); 3.60 (s, 3H); 3.55 (m, 2H); 3.31 (m, 1H); 3.20 (m, 1H); 1.85 (m, 1H); 1.65-1.32 (m, 5H); 1.22 (m, 9H); ³¹p-NMR (CDCl₃): δ = 20.05 ppm (s): δ = 19.90 ppm (s).
f) Die Verseifung der Estergruppen in 2-Phosphono-3-(4-hydroxybutyl)-bernsteinsäure-methyl-trie thylester wurde analog zum Beispiel 1 c) durchgeführt und ergab die freie 2-Phosphono-3-(4-hydroxybutyl)-bernsteinsäure, die mittels 1 N NaOH in das Tetranatriumsalz überführt wurde. C,H-Analyse: C₈H₁₁O₈PNa₄ x 2.5 H₂O (MG = 403) ber. C 23.82; H 3.97; P 7.69; gef. C 23.75; H 3.90; P 7.69.

### Beispiel 4

### 2-Phosphono-3-(3-hydroxypropyl)-bernsteinsäure

a) Analog zum Beispiel 3 a) wurde aus δ-Valerolacton 5-Hydroxyvaleriansäuremethylester hergestellt, der ohne zusätzliche Reinigung weiter umgesetzt wurde.
b) Die Einführung der Silylschutzgruppe für die Hydroxylgruppe in 5-Hydroxyvaleriansäuremethylester erfolgte analog zum Beispiel 3 b) und lieferte 5-tert.-Butyldimethylsilyloxyvaleriansäuremethylester als hellgelbes Öl. ¹H-NMR (CDCl₃): δ = 3.67 ppm (s, 3H); 3.54 (t, 2H); 1.95 (t, 2H); 1.57 (m, 2H); 1.48 (m, 2H); 0.82 (s, 9H); 0.01 (s, 6H).
c) Die Tieftemperaturbromierung von 5-tert.-Butyldimethylsilyloxyvaleriansäuremethylester, die in Analogie zum Beispiel 2 a) erfolgte, lieferte den 2-Brom-5-tert.-butyldimethylsilyloxy-Valeriansäuremethyles ter als gelbes Öl. ¹H-NMR (DMSO-d⁶): δ = 4.5 ppm (dd, 1H); 3.67 (s, 3H); 3.54 (t, 2H); 1.95 (m, 2H); 1.48 (m, 2H); 0.80 (s, 9H); 0.01 (s, 6H).
d) Die Alkylierung von Phosphonoessigsäuretriethylester mit 2-Brom-5-tert.-butyldimethylsilyloxyvaleriansäuremethylest er, die analog zum Beispiel 1 b) durchgeführt wurde, ergab den 2-Phosphono-3-(3-tert.-butyldimethylsilyloxypropyl)-berns teinsäure-methyl-triethylester als farbloses Öl. ¹H-NMR-(DMSO-d⁶) (Diastereomeren-Gemisch 1/1); δ = 4.0 ppm (m, 6H); 3.58 (s, 3H); 3.47 (m, 2H); 3.17 (m, 1H); 2.83 (m, 1H); 1.38 (m, 2H); 1.13 (m, 2H + 9H); 0.81 (s, 9H); 0.01 (s, 6H); ³¹p-NMR (DMSO-d⁶): δ = 24.72 ppm (s); δ = 24.21 ppm (s).
e) Die Entfernung der Silylschutzgruppe aus 2-Phosphono-3-(3-tert.-butyldimethylsilyloxypropyl)-berns teinsäure-methyl-triethylester erfolgte analog zum Beispiel 3 e) und lieferte 2-Phosphono-3-(3-hydroxypropyl)-bernsteinsäure-methyl-tri ethylester ¹H-NMR (DMSO-d⁶) (Diastereomeren-Gemisch 1/1): δ = 4.38 ppm (t, 1H; OH); 4.05 (m, 6H); 3.5 (s, 3H); 3.30 (m, 2H); 3.15 (m, 1H); 3.0 (m, 1H); 1.62-1.20 (m, 4H); 1.17 (m, 9H).
f) Die Hydrolyse der Estergruppen in 2-Phosphono-3-(3-hydroxypropyl)-bernsteinsäure-methyl-tri ethylester wurde analog zum Beispiel 1 c) durchgeführt und ergab die freie 2-Phosphono-(3-hydroxypropyl)-bernsteinsäure, die mittels 1 N NaOH in das Trinatriumsalz überführt wurde. C,H-Analyse: C₇H₁₀O₈Na₃P x0.5 H₂O (331) ber. C 25.39; H 3.75; P 9.37; gef. C 25.27; H 3.51; P 9.60

### Beispiel 5

### 2-Phosphono-3-methoxymethylbernsteinsäure

a) 7.19 g (30 mmol) 2-Brommethylfumarsäuredimethylester (J. Org. Chem. 34, 1228 (1969) wird zu einer Lösung aus 33 mmol Natrium in 100 ml Methanol gegeben und 12 h am Rückfluß erhitzt. Das Methanol wird abdestilliert und der Rückstand über Kieselgel (Laufmittel Diethylether/Heptan 1:4) gereinigt. Man erhält so 2.82 g 2-Methoxymethylfumarsäuredimethylester.
b) Zu einer Lösung aus 5 mmol NaH in Toluol gibt man 900 mg (5 mmol) 2-Methoxymethylfumarsäuredimethylester und tropft 0.55 g (5 mmol) Phosphorigsäurediethylester langsam zu. Nach 1 h bei RT wird eingeengt und die Mischung durch Chromatographie über Kieselgel (Laufmittel Aceton/Toluol 1:1) gereinigt. Ausbeute 0.9 g (60 %) farbloses Öl. 800 mg (2.5 mmol) 3-Methoxymethyl-2-diethylphosphono-bernsteinsäure dimethylester wird in 70 ml 6 N HCI für 6 h bei 140°C erhitzt. Die Lösung wird eingeengt und der Rückstand aus Wasser/Aceton ausgefällt. Man erhält 540 mg (79 %) amorphes, weißes Pulver, dessen Struktur NMR- und massenspektrosko-pisch bestätigt wurde. Fp. 144°C (Zers.).

### Beispiel 6

### 1-Phosphono-1,2-cyclohexan-dicarbonsäure

a) Die Lösung von 1.2 g (3.3 mMol) des im Beispiel 3 e) hergestellten 2-Phosphono-3-(4-hydroxybutyl)-bernsteinsäureesters und 0.73 ml (5.0 mMol) Triethylamin in 20ml Diethylether wird mit 0.3 ml (3.9 mMol) Methansulfonsäurechlorid in 5 ml Diethylether bei Raumtemperatur tropfenweise versetzt und anschließend die Reaktionsmischung 30 min gerührt. Danach wird der ausgefallene Niederschlag abfiltriert, die Etherlösung nacheinander mit 10 ml verdünnter Salzsäure, 20 ml gesättigter Natriumhydrogencarbonat-Lösung und 20 ml Wasser gewaschen und über Natriumsulfat getrocknet. Nach Abziehen des Lösungsmittels erhält man 1.2 g 2-Phosphono-3-(4-methan- sulfonyloxybutyl)-bernsteinsäure-methyl-triethyl-ester. ¹H-NMR (CDCl₃): δ = 4.1 ppm (m, 6H + 2H); 3.65 (s, 3H); 3.48 - 3.03 (m, 2H); 2.92 (s, 3H); 1.85 (m, 2H); 1.78 - 1.40 (m, 4H); 1.22 (m, 9H).
b) Zur Lösung von 1.1 g (2.5 mMol) 2-Phosphono-3-(4-methansulfonyloxy-butyl)-bernsteinsäure-methyl-triethylester in 5 ml trockenem Dimethylformamid gibt man 60 mg (2.5mMol) Natriumhydrid und erhitzt dann die Reaktionsmischung 2 h bei 50 °C. Anschließend dampft man das Lösungsmittel i.V. ab, nimmt den Rückstand in 5ml gesättigter Ammoniumchloridlösung auf und extrahiert die entstandene wäßrige Lösung zweimal mit je 10 ml Essigsäureethylester. Nach Trocknen der vereinigten organischen Phasen über Natriumsulfat und Abziehen des Lösungsmittels wird das Rohprodukt säulenchromatographisch an Kieselgel gereinigt (Laufmittel: Essigsäureethylester). Man erhält so 520 mg 1-Diethylphosphono-2-methoxycarbonyl-cyclohexancarbonsäureethylester. ¹H-NMR (CDCl₃) (Diastereomerengemisch 1/3): δ = 4.1 ppm (m, 6H); 3.60 (s, 3H); 3.40 (m, 1H); 2.11 (m, 2H); 1.86 (m, 2H); 1.61 - 1.35 (m, 4H); 1.22 (m, 9H). ³¹P-NMR (CDCl₃): δ = 23.5 ppm (s); δ = 22.97 ppm (s).
c) Die Hydrolyse der Estergruppen in 1-Diethylphosphono-2-methoxycarbonyl-cyclohexan-carbonsäureethylester wurde analog zum Beispiel 1 c) durchgeführt und ergab die freie 1Phosphono-1,2-cyclohexan-dicarbonsäure, die mittels 1N NaOH in das Trinatriumsalz überführt wurde. ¹H-NMR (D₂O): δ = 2.80 ppm (m, 1H); 2.45 (m, 1H); 2.02 (m, 1H); 1.9 - 1.29 (m, 6H). ³¹P-NMR (D₂O): δ = 18.05 ppm (s).

### Versuchsprotokoll

### Inhibierung der nichtstimulierten Knochenresorption durch die Bestimmung der [³H]-Tetracyclin-Ausscheidung im Urin:

Von Geburt an erhalten Ratten zweimal wöchentlich eine Injektion mit steigenden Mengen einer Lösung aus 3.7 x 10⁵ Bq/ml (10 µCi/ml) [7-³H]-Tetracyclin ([³H]TC); New England Nuclear, Boston, MA) der spezifischen Aktivität 679 mCi/mmol. Das Volumen pro Injektion wird von 50 µl/Woche auf 250 µl in der fünften Woche gesteigert und für eine weitere Woche beibehalten. Die Gesamtmenge an appliziertem [³H]TC betrug 20 µCi pro Ratte. 51 Tage alte Ratten werden in individuelle Stoffwechselkäfige umgesetzt und erhalten eine Gruppenfütterung mit Futter, das 0.5 % Ca und 0.35 % P enthält. Dieses Futter wurde durch Zusatz von entsprechenden Mengen Calciumglucagonat und neutralem Natriumphosphat aus Futter mit niedrigem Calcium- und Phosphatgehalt (SODI 2134, Klingenthalmühle) hergestellt. Während der gesamten Versuchsdauer erhielten die Tiere Aqua dest. ad libitum. Nach 10 Tagen wurde mit dem Sammeln des 24 h Urins begonnen. Die erhielten das Futter täglich um 11 Uhr. Zu dieser Zeit wurde auch der Urin gesammelt. Die Substanzen wurden täglich in zwei s.c. Injektionen verabreicht (8 Uhr bzw. 17 Uhr). Das ³H-TC im Urin wurde durch Flüssigkeitsszintillation bestimmt, indem zu 1 ml Urin 10 ml des Szintillators Pico-Fluor 30 (packard International, Zürich, Schweiz) zugesetzt wurde.

| **Verbindung** | **Bsp.** | **Dosis** | **Resorp.Inhib.** |
|---|---|---|---|
| 2-Phosphono-3-(4-hydroxybutyl)-bernsteinsäure | 3 | 2 x 100 mg/kg | 27 % |
| 2-Phosphono-3-(phenylmethyl)-bernsteinsäure | BV 10 | 2 x 200 mg/kg | 47 % |
| 2-Phosphono-3-(cyclohexylmethyl)-bernsteinsäure | 1 | 2 x 200 mg/kg | 64 % |

## Patentansprüche

1. Verbindungen der Formel I 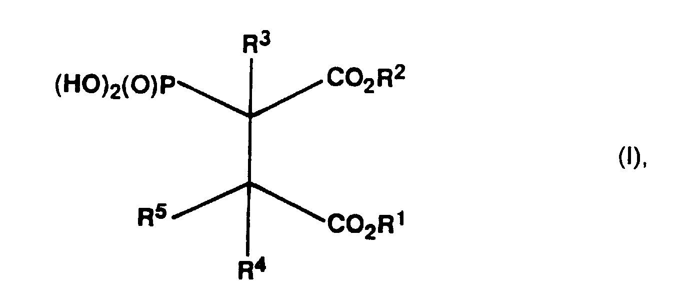 in der
R¹, R² unabhängig voneinander jeweils Wasserstoff, niederes Alkyl, Cycloakyl oder Arylmethyl sein können,
R³, R⁴ unabhängig voneinander jeweils Wasserstoff, niederes Alkyl, oder eine der Gruppen -OR⁶ oder -NR⁷R⁸ sein können, oder zusammen mit den Atomen, an die sie gebunden sind, einen fünf- bis siebengliedrigen carbocyclischen oder einen eins bis zwei Heteroatome enthaltenden heterocyclischen Ring bilden können,
R⁵ niederes Alkenyl, Cycloalkyl, Cycloalkenyl, monocyclisches Arylalkyl, bicyclisches Aryl, Biaryl, oder eine Gruppe der Formeln a) oder b) bedeutet, oder zusammen mit R⁴ und dem Kohlenstoffatom an dem es gebunden ist, einen gegebenenfalls substituierten fünf- bis siebengliedrigen carbocyclischen oder heterocyclischen Ring bildet,
a) R⁹-X-alk- 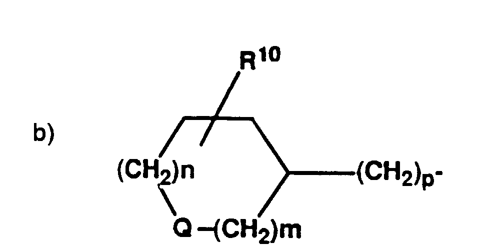
R⁶ Wasserstoff, niederes Alkyl oder Arylmethyl bedeutet,
R⁷, R⁸ unabhängig voneinander jeweils Wasserstoff oder niederes Alkyl bedeuten, oder zusammen mit dem Stickstoffatom, an das sie gebunden sind, einen fünf- bis sechsgliedrigen heterocyclischen Ring bilden,
R⁹ Wasserstoff, niederes Alkyl, niederes Alkenyl, Cycloalkyl, Cycloalkenyl, mono- oder bicyclisches Aryl, Biaryl bedeutet,
R¹⁰ eine Gruppe der Formel a) bedeutet,
X Valenzstrich, Sauerstoff oder Schwefel bedeutet,
Q Sauerstoff, Schwefel oder Stickstoff bedeutet,
alk einen Valenzstrich, eine Methylen-. eine gesättigte oder ungesättigte Alkylenkette mit 2 - 6 Kohlenstoffatome bedeutet,
n = 0-3,
m = 0- 2 und
p = 0 - 5 bedeutet,
sowie deren pharmakologisch unbedenkliche Salze und optische Isomeren, wobei für den Fall, daß R³ Wasserstoff oder C₁ -C₃ Alkyl und R⁴ Wasserstoff bedeutet, R⁵ nicht Wasserstoff, Hydroxy, Methoxy, C₁ -C₃ Alkyl oder Aryl sein darf,
und für den Fall, daß
X Sauerstoff oder Schwefel und R⁴ eine der Gruppen OR⁶ oder NR⁷R⁸ bedueten, alk kein Valenzstrich sein darf, und mit Ausnahme der Verbindungen
2-Phosphono-3-(pyrrolidin-2-yl )-bernsteinsäure,
2-Phosphono-3-(pyrrolidin-3-yl )-bernsteinsäure,
2-Phosphono-3-(pyrrolidin-2-yl-methyl)-bernsteinsäure,
2-Phosphono-3-(pyrrolidin-3-yl-methyl)-bernsteinsäure,
2-Phosphono-3-[2-(pyrrolidin-2-yl)ethyl]-bernsteinsäure,
2-Phosphono-3-[2-(pyrrolidin-3-yl)ethyl]-bernsteinsäure,
2-Phosphono-3-[4-(pyrrolidin-2-yl)butyl]-bernsteinsäure.

2. Arzneimittel, enthaltend mindestens eine Verbindung der Formel I gemäß Anspruch 1 neben üblichen Träger- und Hilfsstoffen.

3. Verwendung von Verbindungen der Formel I gemäß Anspruch 1 zur Herstellung von Arzneimitteln zur Behandlung von Calciumstoffwechselstörungen.

## Claims

1. Compounds of formula I, 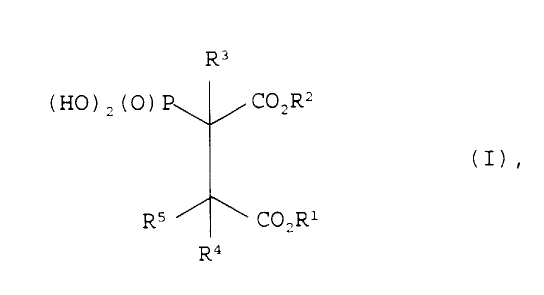 in which
R¹, R² can be, independently of each other, hydrogen, lower alkyl, cycloalkyl or arylmethyl,
R³, R⁴ can be, independently of each other, hydrogen, lower alkyl or one of the groups -OR⁶ or -NR⁷R⁸ or, together with the atoms to which they are bound, can form a five-to seven-membered carbocyclic ring or a heterocyclic ring containing one to two heteroatoms,
R⁵ denotes lower alkenyl, cycloalkyl, cycloalkenyl, monocyclic arylalkyl, bicyclic aryl, biaryl or a group of formula a) or b) or, together with R⁴ and the carbon atom to which it is bound, forms a five- to seven-membered carbocyclic or heterocyclic ring which can be substituted if desired,
a) R⁹-X-alk- 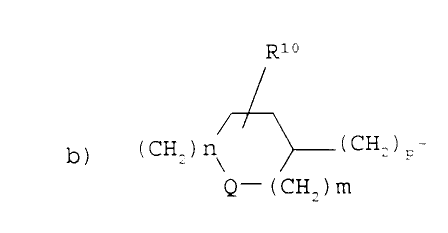
R⁶ denotes hydrogen, lower alkyl or arylmethyl,
R⁷, R⁸ denote, independently of each other, hydrogen or lower alkyl, or together with the nitrogen atom to which they are bound, form a five- to six-membered heterocyclic ring,
R⁹ denotes hydrogen, lower alkyl, lower alkenyl, cycloalkyl, cycloalkenyl, monocyclic or bicyclic aryl, biaryl,
R¹⁰ denotes a group of formula a),
X denotes a valency dash, oxygen or sulphur,
Q denotes oxygen, sulphur or nitrogen,
alk denotes a valency dash, a methylene chain, a saturated or unsaturated alkylene chain with 2 - 6 carbon atoms,
n = 0 - 3
m = 0 - 2 and
p = 0 - 5
as well as pharmacologically acceptable salts thereof and optical isomers wherein in the case that
R³ denotes hydrogen or C₁-C₃ alkyl and R⁴ denotes hydrogen, R⁵ may not be hydrogen, hydroxy, methoxy, C₁-C₃ alkyl or aryl,
and in the case that
X denotes oxygen or sulphur and R⁴ denotes one of the groups OR⁶ or NR⁷R⁸, alk may not be a valency dash, and with exception of the following compounds:
2-Phosphono-3- (pyrrolidin-2-yl) -succinic acid
2-Phosphono-3- (pyrrolidin-3-yl) -succinic acid
2-Phosphono-3- (pyrrolidin-2-yl-methyl) -succinic acid
2-Phosphono-3- (pyrrolidin-3-yl-methyl) -succinic acid
2-Phosphono-3- [2- (pyrrolidin-2-yl) ethyl] -succinic acid
2-Phosphono-3- [2- (pyrrolidin-3-yl)ethyl] -succinic acid
2-Phosphono-3- [4- (pyrrolidin-2-yl)butyl] -succinic acid.

2. Pharmaceutical agent containing at least one compound of the formula I as claimed in claim 1 in addition to the usual carriers and auxiliary substances.

3. Use of compounds of formula I as claimed in claim 1 for the production of pharmaceutical agents for treating disorders of calcium metabolism.

## Revendications

1. Composés de formule I 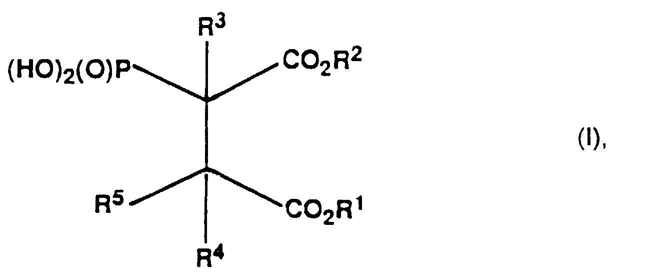 dans laquelle
R¹, R² peuvent représenter chacun, indépendamment, un atome d'hydrogène, un groupe alkyle inférieur, un groupe cycloalkyle ou arylméthyle,
R³, R⁴ peuvent représenter chacun, indépendamment, un atome d'hydrogène, un groupe alkyle inférieur ou un groupe -OR⁶ ou -NR⁷R⁸, ou peuvent former, avec les atomes auxquels ils sont liés, un carbocycle ayant cinq à sept maillons ou un hétérocycle contenant un à deux hétéroatomes,
R⁵ représente un groupe alcényle inférieur, cycloalkyle, cycloalcényle, arylalkyle monocyclique, aryle bicyclique, biaryle, ou un groupe de formule a) ou b), ou forme, conjointement avec R⁴ et l'atome de carbone auquel il est lié, un noyau carbocyclique ou hétérocyclique ayant cinq à sept maillons, éventuellement substitué,
a) R⁹-X-alk- 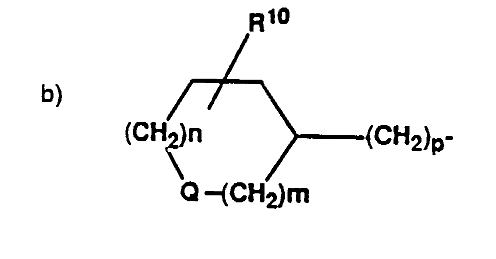
R⁶ représente un atome d'hydrogène, un groupe alkyle inférieur ou arylméthyle,
R⁷, R⁸ représentent chacun, indépendamment, un atome d'hydrogène ou un groupe alkyle inférieur, ou forment, avec l'atome d'azote auquel ils sont liés, un hétérocycle ayant cinq à six maillons,
R⁹ représente un atome d'hydrogène, un groupe alkyle inférieur, alcényle inférieur, cycloalkyle, cycloalcényle, aryle mono- ou bicyclique, biaryle,
R¹⁰ représente un groupe de formule a),
X représente une valence libre, un atome d'oxygène ou de soufre,
Q représente un atome d'oxygène, de soufre ou d'azote,
alk représente une valence libre, un groupe méthylène, une chaîne alkylène saturée ou insaturée, comprenant 2-6 atomes de carbone,
n = 0-3,
m = 0-2 et
p = 0-5,
ainsi que leurs sels pharmacologiquement acceptables et leurs isomères optiques, étant entendu que, dans le cas où R³ représente un atome d'hydrogène ou un groupe alkyle en C₁-C₃ et R⁴ représente un atome d'hydrogène, R⁵ ne doit pas représenter un atome d'hydrogène, un groupe hydroxy, méthoxy, alkyle en C₁ -C₃ ou aryle,
et dans le cas où
X représente un atome d'oxygène ou de soufre et R⁴ représente l'un des groupes -OR⁶ ou -NR⁷R⁸, alk ne doit pas représenter une valence libre, et à l'exception des composés
acide 2-phosphono-3-(pyrrolidin-2-yl)-succinique,
acide 2-phosphono-3-(pyrrolidin-3-yl)-succinique,
acide 2-phosphono-3-(pyrrolidin-2-yl-méthyl)-succinique,
acide 2-phosphono-3-(pyrrolidin-3-yl-méthyl)-succinique,
acide 2-phosphono-3-[2-(pyrrolidin-2-yl)éthyl]-succinique,
acide 2-phosphono-3-[2-(pyrrolidin-3-yl)éthyl]-succinique,
acide 2-phosphono-3-[4-(pyrrolidin-2-yl)butyl]-succinique.

2. Médicament contenant au moins un composé de formule I selon la revendication 1, en plus des supports et adjuvants usuels.

3. Utilisation de composés de formule I selon la revendication 1, pour la préparation de médicaments destinés au traitement des troubles du métabolisme calcique.
